# FASCICULE DE BREVET EUROPEEN

(11) **EP 2 575 644 B1**
(45) Date de publication et mention de la délivrance du brevet: **22.10.2014**
(21) Numéro de dépôt: 11728353.1
(22) Date de dépôt: 03.06.2011
(51) Int. Cl.: A61B 17/16, A61B 17/88

(54) **INSTRUMENTS POUR LA CHIRURGIE RACHIDIENNE MINI-INVASIVE ET LEURS APPLICATIONS**
INSTRUMENTE FÜR DIE MINIMALINVASIVE WIRBELSÄULENCHIRURGIE UND DEREN VERWENDUNG
INSTRUMENTS FOR MINI-INVASIVE SPINAL COLUMN SURGERY AND USES THEREOF

(30) Priorité: 04.06.2010 FR 1054383
(43) Date de publication de la demande: 10.04.2013
(73) Titulaire: Spineart SA, 1217 Meyrin (CH)
(72) Inventeur: LEVIEUX, Jérôme, Laguna Beach, CA 92651 (US)
(74) Mandataire: Cabinet Plasseraud
(86) Numéro de dépôt international: PCT/FR2011/051267
(87) Numéro de publication internationale: WO 2011/151606

(56) Documents cités:
- WO-A1-00/48521
- US-A1- 2005 080 422
- US-A1- 2007 038 034

## Description

La présente invention concerne des instruments pour la chirurgie rachidienne mini-invasive et leurs applications.

Les nombreuses pathologies du rachis sont traitées de différentes manières et l'une d'entre-elle est la chirurgie. En fonction des indications, différents types de chirurgies sont proposés aux patients. L'une d'entre-elles est l'ostéosynthèse. L'ostéosynthèse consiste à placer des vis dans les pédicules vertébraux et de relier ces vis par des barres ou des plaques afin de bloquer le segment malade. Une variante consiste à relier les vis par des barres (ou systèmes) souples ou semi-rigides pour stabiliser le segment malade sans le bloquer. Dans tous ces cas le chirurgien place au préalable des vis dans les pédicules vertébraux.

Depuis peu de temps, ces techniques sont effectuées de façon « mini-invasive », c'est-à-dire que le chirurgien pratique de petites incisions et place les vis au travers de tubes (en aveugle).

En chirurgie mini-invasive, la mise en place des vis pédiculaires se fait au travers de tubes. Actuellement le chirurgien repère l'entrée du pédicule avec un système constitué d'un trocart et d'une petite broche nommé "Jamshidi" du nom de l'inventeur. Lorsque l'entrée du pédicule est repérée le chirurgien enfile ce qui est nommé un câble de Kirschner, du nom original "Kirschner wire" encore appelée un "K-Wire" au travers du trocart et l'enfonce dans le pédicule sous contrôle radiographique.

Lorsque le K-wire est en place le chirurgien enfile autour du K-wire une série de tubes dilatateurs de diamètres croissants afin d'écarter les muscles, puis il retire tous les tubes sauf le plus gros. La vis pédiculaire et son porte implant sont canulés. Ils sont alors implantés, en les enfilant sur le K-Wire. Cela permet de s'assurer que la vis est bien implantée dans le pédicule puisqu'elle est guidée par le K-wire planté dans le pédicule.

L'avantage de ce type de chirurgie est que l'incision est plus petite que dans la technique invasive classique (dite « à ciel ouvert »), et laisse peu de cicatrices. En outre les désinsertions musculaires sont presque nulles. Au total, le patient présente de meilleures suites opératoires en particulier des douleurs post-opératoires réduites.

Mais cette technique se développe très lentement car elle reste aujourd'hui plus délicate à pratiquer que la technique invasive classique.

En outre elle présente plusieurs inconvénients :
- les implants et les instruments doivent être canulés, ce qui augmente leur prix de revient;
- le K-wire est à usage unique, ce qui augmente aussi le coût;
- le K-wire est un outil dangereux et qu'il faut donc manipuler avec précaution. Par exemple lorsque l'on implante une vis, il faut opérer sous contrôle radiographique en continu par rayons X pour vérifier que le K-wire ne soit pas entraîné par la vis.

Un autre inconvénient est donc que cette technique augmente l'irradiation du patient et du personnel soignant

Il serait souhaitable de placer de façon sûre des vis faciles à fabriquer et bon marché, notamment non canulées, dans les pédicules vertébraux par chirurgie mini-invasive.

Pour cela il serait souhaitable de disposer d'un instrument ou d'un ensemble d'instruments simples, faciles à fabriquer et bon marché.

Il serait aussi souhaitable de permettre au chirurgien de placer de façon sûre des vis dans les pédicules vertébraux sans les inconvénients liés aux systèmes existants et notamment en limitant le nombre de contrôles radiographiques.

Le K-wire étant un instrument dangereux, à manipuler avec précaution; il serait souhaitable d'éviter son utilisation.

US 2005/080422 décrit un ensemble d'instruments chirurgicaux pour utilisation avec des implants spinaux expansibles. L'ensemble d'instruments comprend notamment un instrument permettant l'assemblage et la rétraction de l'implant, cet instrument étant configuré pour assembler un implant et le rétracter ou réduire sa hauteur.

Or après de longues recherches la demanderesse a élaboré un nouveau système pour la chirurgie rachidienne mini-invasive, donnant toute satisfaction.

L'invention consiste à remédier aux inconvénients de l'art antérieur en utilisant un gros tube de dilatation, habituellement le dernier tube de dilatation ou « tube guide » pour guider la vis dans le pédicule. La position et l'orientation de ce tube doivent être précises. C'est pourquoi il a été imaginé un perforateur pédiculaire de type classique, comme ceux utilisés dans la chirurgie rachidienne invasive standard, mais modifié de façon à placer et à orienter le tube guide de manière optimale et in fine à placer et à orienter de manière optimale une vis pédiculaire.

Par ailleurs, le tube guide présente avantageusement des dents, ou des pointes ou d'autres types d'accroche afin qu'il soit stable une fois au contact de l'os.

Ainsi on peut notamment se dispenser de l'utilisation de K-wire, de vis canulées et même des trocarts « Jamshidi » et réduire ainsi les coûts et les inconvénients de cette chirurgie.

C'est pourquoi la présente demande a pour objet ensemble comprenant un perforateur pédiculaire comprenant une poignée et une tige terminée à l'opposé de la poignée par une extrémité ayant une forme perforante (ci-après dénommée "pointe"), caractérisé en ce que la tige comprend un dispositif saillant de centrage pour surface cylindrique prévu dans une zone de la tige opposée à la poignée et un tube guide creux comme décrit ci-aprés. Un perforateur pédiculaire est communément appelé « pedicle probe » en anglais.

A noter que dans la présente demande, classiquement l'article indéfini "un" doit être considéré comme un pluriel générique (signification de "au moins un" ou encore "un ou plusieurs"), sauf lorsque le contexte montre le contraire (1 ou "un seul"). Ainsi, par exemple, lorsque l'on dit ci-dessus que l'on prévoit un dispositif saillant de centrage, il s'agit de prévoir un ou plusieurs dispositifs saillants de centrage.

Dans la présente demande et dans ce qui suit, par " un dispositif saillant de centrage " on entend un dispositif maintenant la tige en position axiale dans un cylindre et en même temps capable de laisser la tige coulisser dans ce cylindre. La tige peut coulisser dans ce cylindre à frottement doux, c'est-à-dire qu'un jeu limité est prévu entre le dispositif de centrage et le cylindre pour un bon centrage. Ce dispositif de centrage est installé en saillie sur la tige.

Le dispositif de centrage peut être fin ou massif. Un dispositif de centrage fin comprend par exemple des baguettes ou des ailettes ou une combinaison d'une bague et de baguettes ou d'ailettes. Un dispositif de centrage massif comprend par exemple un cylindre allongé ou un cylindre allongé en forme de diabolo ou de navette. On comprend que le fait d'être massif ne l'empêche pas d'être creux.

Un dispositif de centrage fin comprend par exemple deux ou plusieurs ensembles de baguettes ou d'ailettes, espacés les uns des autres. Un dispositif de centrage massif sera de préférence unique.

Pour un bon centrage, le dispositif de centrage, lorsqu'il est unique, est prévu dans une zone de la tige opposée à la poignée, donc proche de la pointe. Il est suffisamment long pour que la tige et le tube soit coaxiaux.

Il est alors prévu dans une zone de la tige allant de 1 cm à 25 cm de l'extrémité de la pointe, de préférence de 2 cm à 20 cm de l'extrémité de la pointe, notamment de 3 cm à 15 cm de l'extrémité de la pointe, tout particulièrement de 4 cm à 10 cm de l'extrémité de la pointe.

Plusieurs dispositifs de centrage seront prévus dans une zone de la tige allant de 1 cm à 25 cm de l'extrémité de la pointe, de préférence de 2 cm à 20 cm de l'extrémité de la pointe, notamment de 3 cm à 15 cm de l'extrémité de la pointe, tout particulièrement de 4 cm à 10 cm de l'extrémité de la pointe.

On pourra par exemple prévoir 4, 3 et avantageusement 2 dispositifs de centrage.

Un dispositif de centrage aura une longueur de 20 mm à 250 mm, de préférence de 30 à 200 mm, notamment de 50 à 150 mm, tout particulièrement de 60 à 100 mm. On évaluera cette longueur par la distance entre les projections orthogonales sur la tige des points extrêmes du dispositif de centrage. Pour un cylindre, il s'agira de la longueur du cylindre.

Dans des conditions préférentielles de mise en oeuvre de l'invention, le diamètre intérieur du cylindre ci-dessus capable de laisser la tige et son dispositif de centrage coulisser est d'environ 14,5 mm, par exemple de 12 à 16 mm.

La tige peut avoir par exemple une longueur de 80 à 400 mm , de préférence de 100 à 300 mm notamment de 120 à 250 mm, tout particulièrement de 150 à 220 mm.

De préférence, la tige aura une longueur de 100 à 300 mm avec un dispositif de centrage d'une longueur de 3 cm à 20 cm, et notamment la tige aura une longueur de 120 à 250 mm avec un dispositif de centrage d'une longueur de 5 cm à 15 cm. Dans des conditions préférentielles de mise en oeuvre, ces dispositifs de centrage peuvent coulisser dans un cylindre ci-dessus de diamètre intérieur de 12 à 16 mm.

En général une tige comprenant un dispositif saillant de centrage aura (sauf pour la pointe) un diamètre externe de 2 à 12 mm, de préférence de 3 à 10 mm, notamment de 4 à 9 mm, tout particulièrement de 5 à 8 mm.

La tige sera habituellement pleine.

Le dispositif saillant de centrage sera une pièce rapportée sur la tige ou monobloc avec la tige. Si le dispositif saillant de centrage est de préférence solidaire de la tige c'est-à-dire tourne en même temps que la tige, le dispositif saillant de centrage peut aussi tourner séparément de la tige qui lui sert alors d'axe de rotation.

La tige d'un perforateur pédiculaire de l'invention peut être réalisée en tout matériau adapté à son utilisation par exemple en polymère allié au carbone, comme en polymère allié de matériaux radio opaques et en particulier peut être réalisée en Titane ou alliage de Titane ou de préférence en acier inox.

Les dispositifs saillants de centrage de l'invention peuvent être réalisées en tout matériau polymère comme en polymère allié et en particulier en Titane ou alliage de Titane ou de préférence en acier inox.

Toutes les caractéristiques d'un perforateur pédiculaire de l'invention ci-dessus peuvent être combinées entre elles.

La présente invention a également pour objet un procédé de fabrication de perforateurs pédiculaires tels que définis ci-dessus caractérisé en ce que l'on procède à un usinage ou un forgeage de la tige, à l'usinage du centreur, puis à l'installation d'une poignée. Selon un autre procédé, on rapporter un centreur sur la tige avant ou après installation d'une poignée.

Les perforateurs pédiculaires objets de la présente invention peuvent être utilisés comme suit:
- on réalise un avant-trou à l'aide d'une pointe carrée guidée par des contrôles aux rayons X,
- on enfile sur cette pointe carrée des tubes dilatateurs de diamètres croissant, puis on les retire tous sauf le plus gros tube que l'on maintient au contact de l'os,
- on insère alors le perforateur pédiculaire de la présente invention dans le gros tube. La pointe du perforateur pédiculaire est ainsi guidée sûrement vers l'avant-trou ; on utilise alors le perforateur classiquement pour percer le pédicule,
- une fois le pédicule percé précisément sur une profondeur suffisante, le chirurgien retire le perforateur pédiculaire et maintient le tube dans sa position. Il peut effectuer cette opération à la main ou bien en s'aidant d'un bras articulé bloquant,
- on insère alors dans le tube guide une vis pédiculaire. La vis pédiculaire est guidée par le tube guide et elle a une bonne position et une bonne orientation pour entrer dans le trou réalisé par le perforateur pédiculaire.

Rappelons qu'une vis pédiculaire comporte une partie vissante d'une longueur de plusieurs cm, supérieure à 2,5 cm, souvent supérieure à 3 cm, généralement supérieure à 4 cm, et pouvant dépasser 5 cm. Les caractéristiques du perforateur pédiculaire tiennent compte de cette contrainte. Ainsi par exemple, l'extrémité de la tige comportant la forme perforante a une longueur efficace, c'est-à-dire autorisant la perforation, d'une longueur de plusieurs cm.

Le type conventionnel de perforateur étant couramment utilisé, c'est un outil très fiable pour percer un pédicule avec précision.

A la connaissance de la demanderesse, le tube externe ci-dessus est un produit nouveau.

C'est pourquoi la présente demande a aussi pour objet un tube guide creux de diamètre interne de 4 à 20 mm, notamment de 10 mm à 18 mm, et de préférence d'environ 14,5 mm, de diamètre externe de 5 à 22 mm, notamment de 12 mm à 20 mm, et de préférence d'environ 17 mm, et de longueur de 4 à 25 cm, notamment de 8 cm à 18 cm, et de préférence d'environ 14 cm, comportant une extrémité munie de reliefs d'accroche. Les reliefs d'accroche peuvent être notamment des pointes, des dents etc... Le dispositif d'accroche peut aussi être composé de longues et fines vis permettant de visser le tube sur l'os.

De préférence, le tube guide creux aura un diamètre interne de 10 mm à 18 mm, pour un diamètre externe de 12 mm à 20 mm, et une longueur de 8 cm à 18 cm.

Le diamètre interne du tube guide creux comportant une extrémité munie de reliefs d'accroche est prévue pour le guidage d'un perforateur pédiculaire de l'invention puis d'une vis pédiculaire.

Un tube guide creux de l'invention peut être réalisé en tout matériau adapté à son utilisation par exemple en polymère, en polymère allié au carbone, en polymères alliés de matériaux radio opaques et en particulier peut être réalisé en acier inox ou de préférence en Titane ou alliage de Titane.

La présente demande a aussi pour objet un ensemble pour la perforation pédiculaire et la mise en place de vis pédiculaires en chirurgie mini-invasive rachidienne comprenant au moins un perforateur pédiculaire ci-dessus et au moins un tube guide creux ci-dessus, la conformation du perforateur et le diamètre interne du tube guide creux permettant le glissement à frottement doux du perforateur pédiculaire dans le tube guide creux.

Les perforateurs pédiculaires objets de la présente invention possèdent de très intéressantes propriétés et qualités.

Notamment ils permettent grâce à leur zone de guidage de placer et d'orienter le tube guide de manière optimale. Ceci permet alors au tube guide maintenu dans sa position de guider sûrement une vis dans le pédicule vertébral. Des perforateurs (sans dispositif saillant de centrage) sont très utilisés car ils sont très efficaces pour effectuer des visées pédiculaires. De ce fait, les perforateurs objets de la présente invention (avec dispositif saillant de centrage) permettent aux chirurgiens de réaliser des chirurgies mini-invasives rachidiennes en utilisant un outil qu'ils connaissent bien et en évitant les inconvénients liés à l'utilisation de K-wire. Ils sont faciles et rapides à fabriquer et leur coût de fabrication est ainsi faible.

Le chirurgien peut réaliser une chirurgie mini-invasive rachidienne en opérant selon une technique proche de la technique dite « à ciel ouvert ». Ceci présente donc en plus l'intérêt de rendre la chirurgie mini-invasive rachidienne plus facile d'accès pour tous les chirurgiens. De plus grâce à cette technique, le nombre de contrôle aux rayons X est inférieur à celui classiquement pratiqué lors des chirurgies mini-invasives rachidiennes nécessitant l'utilisation de K-wire.

De plus ce dispositif est particulièrement adapté aux techniques mini-invasives.

Ces qualités sont illustrées ci-après. Elles justifient l'utilisation des perforateurs pédiculaires ci-dessus décrits pour la mise en place de vis pédiculaires vertébrales selon la technique mini-invasive et notamment dans l'ostéosynthèse mini-invasive thoraco-lombaire.

C'est pourquoi la présente demande a aussi pour objet une méthode de mise en place de vis pédiculaires vertébrales dans laquelle
- on réalise dans un pédicule vertébral un avant-trou à l'aide d'une pointe,
- on enfile sur cette pointe des tubes dilatateurs de diamètres croissants, puis on les retire tous sauf le plus gros tube (tube guide) que l'on maintient au contact de l'os,
- on insère un perforateur pédiculaire de la présente invention dans le tube guide pour percer le pédicule,
- on retire le perforateur pédiculaire et maintient le tube guide dans sa position,
- on insère alors dans le tube puis dans le trou réalisé par le perforateur pédiculaire la vis pédiculaire qui est guidée par le tube guide.

Les dispositifs ci-dessus étant pour usage chirurgical, la présente demande a aussi pour objet lesdits dispositifs stériles, notamment conditionnés sous emballage préservant leur stérilité.

Les conditions préférentielles de mise en oeuvre des perforateurs pédiculaires ci-dessus décrits s'appliquent également aux autres objets de l'invention visés ci-dessus, notamment aux procédés et méthodes les mettant en oeuvre et pour leur fabrication.

L'invention sera mieux comprise si l'on se réfère aux dessins annexés sur lesquels
- la figure 1 représente une vue en perspective d'un perforateur pédiculaire de la présente invention,
- la figure 2 représente une vue en perspective d'une variante de perforateur pédiculaire de la présente invention,
- la figure 3 représente une vue en perspective d'un tube guide de la présente invention,
- la figure 4 représente une vue en perspective d'un ensemble d'un perforateur pédiculaire et d'un tube guide de la présente invention,
- la figure 5 représente une utilisation d'un ensemble d'un perforateur pédiculaire et d'un tube guide de la présente invention pour percer un pédicule vertébral,
- la figure 6 est une vue partiellement éclatée de la figure 5.

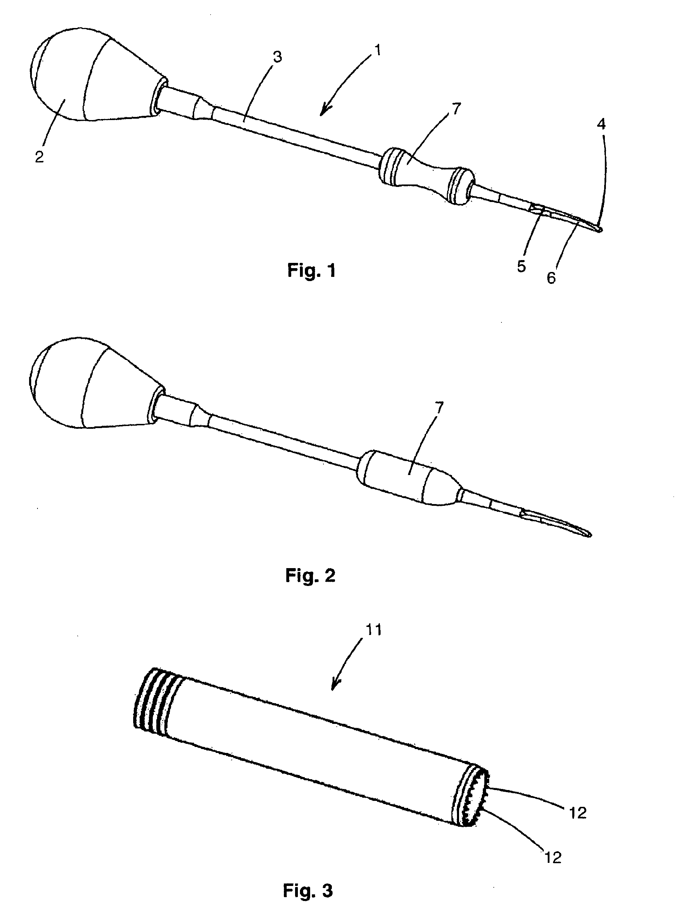

Sur la figure 1 qui est une vue en perspective d'un perforateur pédiculaire 1 de la présente invention, on observe la poignée 2 en forme de poire qui permet de saisir le perforateur pédiculaire 1 et de l'appuyer en lui donnant on mouvement par exemple alternatif de rotation pour perforer un pédicule. La poignée 2 est solidaire d'une tige 3 terminée à l'opposé de la poignée 2 par une extrémité 4 ayant une forme perforante, comportant ici des facettes 5,6. La tige 3 comprend un dispositif de centrage 7 pour surface cylindrique prévu dans une zone de la tige 3 opposée à la poignée 2. Le dispositif de centrage 7 est ici unique, massif et en forme de diabolo.

Le dispositif de centrage 7 maintient la tige 3 en position axiale dans un cylindre et en même temps laisse la tige 3 coulisser dans ce cylindre comme on le verra ci-après. Ce dispositif de centrage 7 est installé en saillie sur la tige 3 car ses diamètres sont supérieurs au diamètre de la tige 3 à l'emplacement où il est fixé. La tige 3 est centrée dans le dispositif de centrage 7. Le dispositif de centrage 7 est ici unique, massif et en forme de diabolo.

Le dispositif de centrage 7 est, dans cette mise en oeuvre, solidaire de la tige 3.

Il est prévu dans une zone de la tige 3 opposée à la poignée 2, donc proche de la pointe 4. Il est suffisamment long pour que la tige 3 et le dispositif de centrage 7 soit coaxiaux.

Dans cet exemple de mise en oeuvre il a une longueur de 5 cm et est prévu dans la zone de la tige 3 allant de 3cm à 8 cm de l'extrémité 4 ayant une forme perforante. Son diamètre maximal est de 14,2 mm environ et son diamètre minimal vers le milieu de sa longueur est de 14 mm environ.

La tige 3 a ici une longueur mesurée entre l'extrémité 4 et l'extrémité opposée, à la sortie de la poignée 2, de 200 mm environ. La tige 3 est pleine (massive).

Sur la majorité de sa longueur, la tige 3 a un diamètre externe constant 6 mm environ. à proximité de son canal d'introduction dans la poignée 2, la tige 3 a un diamètre externe augmenté.

Sur la figure 2, on observe un dispositif de centrage 7 pour surface cylindrique prévu dans une zone de la tige 3 opposée à la poignée 2. Le dispositif de centrage 7 est ici unique, massif et principalement de forme cylindrique, fuselée du côté de l'extrémité 4 ayant une forme perforante.

Sur la figure 3, on observe un tube guide 11. Le tube guide 11 a un diamètre interne de 14,5 mm environ et un diamètre externe de 17,5 mm environ. Ainsi le dispositif de centrage 7 peut coulisser à frottement doux dans le tube pour guider le perforateur pédiculaire 1 pour perforer un pédicule exactement à l'endroit désiré. Le tube 11 est muni à une de ses extrémités de reliefs d'accroche qui sont ici des dents 12.

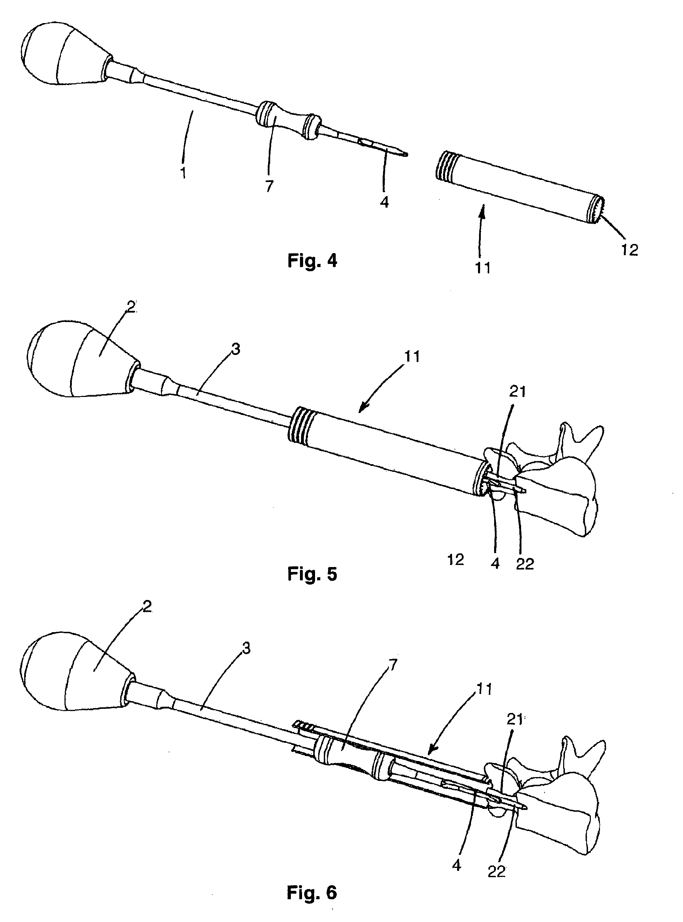

Sur la figure 4, on observe un perforateur pédiculaire 1 en position d'introduction dans un tube guide 11. Ce tube guide dilatateur 11 est le tube guide externe laissé en place après installation des tubes dilatateurs de diamètres croissant

Sur la figure 5, on observe un ensemble d'un perforateur pédiculaire 1 et d'un tube guide 11 de la présente invention en action pour percer un pédicule vertébral 21.

Un perforateur pédiculaire 1 a été introduit dans un tube guide 11. Guidée par le dispositif de centrage 7 (visible sur la figure suivante), l'extrémité 4 ayant une forme perforante est centrée dans le tube guide 11. Celui-ci, grâce à ses extrémités munies de reliefs d'accroche a été bien placé pour viser l'emplacement à percer sans glisser. L'extrémité 4 ayant une forme perforante a été activée en rotation par manipulation de la poignée 2, et a percé le pédicule vertébral 21 à l'emplacement et selon l'angle choisi pour créer un canal 22 dans le pédicule vertébral 21. Après retrait du perforateur pédiculaire 1 et maintien en position du tube guide 11 grâce à ses extrémités munies de reliefs d'accroche, une vis pédiculaire de diamètre de tête adaptée (environ 14 mm) peut être guidée sûrement vers le canal 22 formant un pré trou pour être vissée.

Sur la figure 6, on observe les mêmes éléments que sur la figure 5 et d'autres du fait que la figure est éclatée.

On a également réalisé sur le même principe les cinq perforateurs pédiculaires de dimensions et nature suivantes:

| | 2 | 3 | 4 | 5 | 6 |
|---|---|---|---|---|---|
| Matériau utilisé | Inox | Titane | Radel | Composite Polymère carbone | Polymère |
| Longueur de la tige hors poignée cm | 18,0 | 20 | 15 | 18 | 15 |
| Diamètre extérieur de la tige mm | 5 | 5,5 | 6 | 5 | 7 |
| Nombre de dispositifs de centrage | 1 | 2 | Deux zones de 9mm de long | 3 | 3 |
| Nature du dispositif de centrage | Radel | Titane | Radel | Composite Polymère carbone | Polymère |
| Forme du dispositif de centrage | Cylindre | Cylindre | Diabolo | Cylindre | 6 Ailettes |

## Revendications

1. Un ensemble comprenant un perforateur pédiculaire (1) comprenant une poignée (2) et une tige (3) terminée à l'opposé de la poignée (2) par une extrémité (4) ayant une forme perforante (ci-après dénommée "pointe"), **caractérisé en ce que** la tige (3) comprend un dispositif saillant de centrage (7) pour surface cylindrique prévu dans une zone de la tige (3) opposée à la poignée (2) et un tube guide creux (11) de diamètre interne de 4 à 20 mm, de diamètre externe de 5 à 22 mm et de longueur de 4 à 25 cm, comportant une extrémité munie de reliefs d'accroche (12), le perforateur pédiculaire (1) pouvant coulisser à frottement doux dans ce tube guide creux (11).

2. Un ensemble selon la revendication 1, **caractérisé en ce que** le dispositif de centrage (7) est massif comme un cylindre allongé ou est un cylindre allongé en forme de diabolo ou de navette.

3. Un ensemble selon la revendication 1 ou 2, **caractérisé en ce que** le dispositif de centrage (7) est unique sur la tige (3).

4. Un ensemble selon la revendication 3, **caractérisé en ce que** le dispositif de centrage (7) unique est prévu dans une zone de la tige allant de 2 cm à 20 cm de l'extrémité de la pointe.

5. Un ensemble selon l'une des revendications 1 à 4, **caractérisé en ce que** dispositif de centrage (7) a une longueur de 50 à 150 mm.

6. Un ensemble selon l'une des revendications 1 à 5, utilisable avec un tube guide cylindrique creux, **caractérisé en ce que** les dimensions d'un dispositif de centrage (7) sont telles que le diamètre intérieur d'un cylindre (11) capable de laisser la tige (3) et son dispositif de centrage (7) coulisser est de 12 à 16 mm.

7. Un ensemble selon l'une des revendications 1 à 6, **caractérisé en ce que** la tige (3) a une longueur de 100 à 300 mm.

8. Un perforateur pédiculaire (1) comprenant une poignée (2) et une tige (3) terminée à l'opposé de la poignée (2) par une extrémité (4) ayant une forme perforante (ci-après dénommée "pointe"), **caractérisé en ce que** la tige (3) comprend un dispositif saillant de centrage (7) pour surface cylindrique prévu dans une zone de la tige (3) opposée à la poignée (2), utilisable avec un tube guide cylindrique creux, **caractérisé en ce que** les dimensions d'un dispositif de centrage (7) sont telles que le diamètre intérieur d'un cylindre (11) capable de laisser la tige (3) et son dispositif de centrage (7) coulisser est de 12 à 16 mm.

9. Un perforateur pédiculaire selon la revendication 8, **caractérisé en ce que** la tige (3) a une longueur de 100 à 300 mm.

## Patentansprüche

1. Anordnung bestehend aus
- einem Pediculus-Perforator (1), der einen Griff (2) und eine Stange (3) aufweist, die an ihrem dem Griff (2) gegenüberliegenden Ende (4) eine perforierende Gestalt (nachstehend "Spitze" genannt) hat, **dadurch gekennzeichnet, dass** die Stange (3) eine vorspringende Zentriervorrichtung (7) aufweist, die zum Zentrieren bezüglich einer zylindrischen Oberfläche vorgesehen ist und in einer dem Griff (2) gegenüberliegenden Zone der Stange (3) angeordnet ist, und
- einem hohlen Führungsrohr (11) mit einem Innendurchmesser von 4 bis 20 mm, einem Außendurchmesser von 5 bis 22 mm und einer Länge von 4 bis 25 cm und mit einem Ende, das mit Hakenprofilen (12) versehen ist,
wobei der Pediculus-Perforator (1) mit sanfter Reibung in dem hohlen Führungsrohr (11) gleiten kann.

2. Anordnung nach Anspruch 1, **dadurch gekennzeichnet, dass** die Zentriervorrichtung (7) massiv ist, beispielsweise ein länglicher Zylinder oder ein länglicher Zylinder in Form eines Doppelkegels oder Schiffchens.

3. Anordnung nach Anspruch 1 oder 2, **dadurch gekennzeichnet, dass** es nur eine einzige Zentriervorrichtung (7) an der Stange (3) gibt.

4. Anordnung nach Anspruch 3, **dadurch gekennzeichnet, dass** die einzige Zentriervorrichtung (7) in einer Zone der Stange vorgesehen ist, die sich 2 cm bis 20 cm, gemessen vom Ende der Spitze, erstreckt.

5. Anordnung nach einem der Ansprüche 1 bis 4, **dadurch gekennzeichnet, dass** die Zentriervorrichtung (7) eine Länge von 50 bis 150 mm hat.

6. Anordnung nach einem der Ansprüche 1 bis 5, die mit einem hohlen zylindrischen Führungsrohr verwendbar ist, **dadurch gekennzeichnet, dass** die Abmessungen einer Zentriervorrichtung (7) derart sind, dass der Innendurchmesser eines Zylinders (11), in welchem die Stange (3) und ihre Zentriervorrichtung (7) gleiten kann, von 12 bis 16 mm beträgt.

7. Anordnung nach einem der Ansprüche 1 bis 6, **dadurch gekennzeichnet, dass** die Stange (3) eine Länge von 100 bis 300 mm hat.

8. Pediculus-Perforator (1), der einen Griff (2) und eine Stange (3) aufweist, die an ihrem dem Griff (2) gegenüberliegenden Ende (4) eine perforierende Gestalt (nachstehend "Spitze" genannt) hat, **dadurch gekennzeichnet, dass** die Stange (3) eine vorspringende Zentriervorrichtung (7) aufweist, die zum Zentrieren bezüglich einer zylindrischen Oberfläche vorgesehen ist und in einer dem Griff (2) gegenüberliegenden Zone der Stange (3) angeordnet ist, wobei der Pediculus-Perforator mit einem hohlen zylindrischen Führungsrohr verwendbar ist,
**dadurch gekennzeichnet, dass** die Abmessungen einer Zentriervorrichtung (7) derart sind, dass der Innendurchmesser eines Zylinders (11), in welchem die Stange (3) und ihre Zentriervorrichtung (7) gleiten kann, von 12 bis 16 mm beträgt.

9. Pediculus-Perforator nach Anspruch 8, **dadurch gekennzeichnet, dass** die Stange (3) eine Länge von 100 bis 300 mm hat.

## Claims

1. Kit comprising a pedicle perforator (1) comprising a handle (2) and a shaft (3), the latter terminating opposite the handle (2) with an end (4) having a perforating shape (hereinafter called the "tip"), **characterized in that** the shaft (3) comprises a protruding centering device (7) for a cylindrical surface, provided in a region of the shaft (3) opposite the handle (2) and a hollow guide tube (11) with an internal diameter of 4 to 20 mm, an external diameter of 5 to 22 mm and a length of 4 to 25 cm, having an end provided with fastening protrusions (12), the pedicle perforator (1) being able to slide with minimal friction in this hollow guide tube.

2. Kit according to Claim 1, **characterized in that** the centering device (7) is solid like an elongate cylinder or an hourglass-shaped or shuttle-shaped elongate cylinder.

3. Kit according to either of Claims 1 and 2, **characterized in that** a single centering device (7) is provided on the shaft (3).

4. Kit according to Claim 3, **characterized in that** the single centering device (7) is provided in a region of the shaft at 2 cm to 20 cm from the end of the tip.

5. Kit according to one of Claims 1 to 4, **characterized in that** the centering device (7) has a length of 50 to 150 mm.

6. Kit according to one of Claims 1 to 5, which can be used with a hollow cylindrical guide tube, **characterized in that** the dimensions of a centering device (7) are such that the internal diameter of a cylinder (11) capable of allowing the shaft (3) and its centering device (7) to slide is from 12 to 16 mm.

7. Kit according to one of Claims 1 to 6, **characterized in that** the shaft (3) has a length of 100 to 300 mm.

8. Pedicle perforator (1) comprising a handle (2) and a shaft (3), the latter terminating opposite the handle (2) with an end (4) having a perforating shape (hereinafter called the "tip"), **characterized in that** the shaft (3) comprises a protruding centering device (7) for a cylindrical surface, provided in a region of the shaft (3) opposite the handle (2), which can be used with a hollow cylindrical guide tube, **characterized in that** the dimensions of a centering device (7) are such that the internal diameter of a cylinder (11) capable of allowing the shaft (3) and its centering device (7) to slide is from 12 to 16 mm.

9. Pedicle perforator according to Claim 8, **characterized in that** the shaft (3) has a length of 100 to 300 mm.
